(19) Europäisches Patentamt
European Patent Office
Office européen des brevets

(11) Veröffentlichungsnummer: **0 118 848 B1**

(12) # EUROPÄISCHE PATENTSCHRIFT

(45) Veröffentlichungstag der Patentschrift: **19.02.92**

(51) Int. Cl.5: **C07D 457/12**, A61K 31/48

(21) Anmeldenummer: **84102230.4**

(22) Anmeldetag: **02.03.84**

Die Akte enthält technische Angaben, die nach dem Eingang der Anmeldung eingereicht wurden und die nicht in dieser Patentschrift enthalten sind.

(54) **Neue Ergolin-Derivate, Verfahren zu ihrer Herstellung sowie Verwendung als Arzneimittel.**

(30) Priorität: **14.03.83 DE 3309493**

(43) Veröffentlichungstag der Anmeldung:
**19.09.84 Patentblatt 84/38**

(45) Bekanntmachung des Hinweises auf die Patenterteilung:
**19.02.92 Patentblatt 92/08**

(84) Benannte Vertragsstaaten:
**AT BE CH DE FR GB IT LI LU NL SE**

(56) Entgegenhaltungen:
**EP-A- 21 206**
**EP-A- 48 695**
**EP-A- 74 921**
**GB-A- 2 093 452**

**MERCK-INDEX, 10. Ausgabe, 1983; S. 791**

**ARZNEIMITTELWIRKUNGEN, E.Mitscher, 4. Auflage, 1981; Seiten 255-257, 335-336**

(73) Patentinhaber: **SCHERING AKTIENGESELLSCHAFT Berlin und Bergkamen**
**Müllerstrasse 170/178 Postfach 65 03 11**
**W-1000 Berlin 65(DE)**

(72) Erfinder: **Sauer, Gerhard, Dr.**
**Königsbacher Zeile 47A**
**W-1000 Berlin 28(DE)**
Erfinder: **Haffer, Gregor, Dr.**
**Mörchinger Str. 79**
**W-1000 Berlin 37(DE)**
Erfinder: **Wachtel, Helmut, Dr.**
**Suarezstr. 22**
**W-1000 Berlin 19(DE)**
Erfinder: **Schneider, Herbert-Hans, Dr.**
**Duisburger Str. 20**
**W-1000 Berlin 15(DE)**
Erfinder: **Kehr, Wolfgang, Dr.**
**Biedermannweg 11**
**W-1000 Berlin 19(DE)**

EP 0 118 848 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue Ergolin-Derivate, Verfahren zu ihrer Herstellung und Arzneimittel auf Basis dieser Verbindungen.

Die Erfindung betrifft Ergoline der allgemeinen Formel I

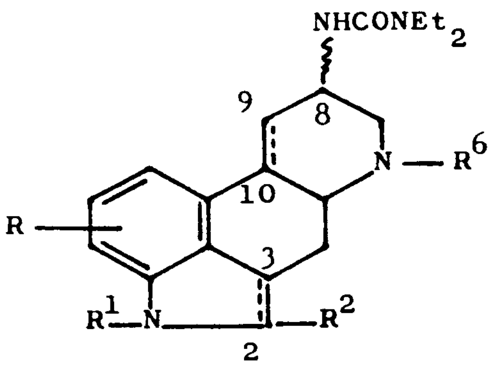

(I)

und deren Säureadditionssalze, worin die 8-ständige Harnstoff-Seitenkette α- oder β-ständig sein kann, $C_2...C_3$ und $C_9...C_{10}$ eine CC-Einfach- oder C=C-Doppelbindung bedeuten und

- R Wasserstoff oder NR'R'', wobei R'R'' für $O_2$, $H_2$, $C_{1-4}$-Dialkyl steht oder gemeinsam mit dem Stickstoffatom einen 3- bis 9-gliedrigen Ring bildet oder R' und R'' jeweils allein für Wasserstoff, $C_{1-4}$-Alkyl oder $C_{1-10}$-Acyl stehen,
- $R^1$ für Wasserstoff, $C_{1-4}$-Alkyl, $C_{1-10}$-Acyl, $C_{6-8}$-Arylsulfonyl, oder $C_{1-4}$-Alkylsulfonyl,
- $R^2$ für Wasserstoff oder Halogen, wenn $C_2...C_3$ eine C=C-Doppelbindung oder Wasserstoff, wenn $C_2...C_3$ eine CC-Einfachbindung darstellt und
- $R^6$ für $C_{1-4}$-Alkyl stehen und, wenn R ein Wasserstoffatom ist, $C_2...C_3$ eine CC-Einfachbindung darstellt.

Die Säureadditionssalze der erfindungsgemäßen Verbindungen leiten sich von physiologisch unbedenklichen Säuren ab. Solche physiologisch unbedenklichen Säuren sind anorganische Säuren, wie beispielsweise Chlorwasserstoffsäure, Salpetersäure, Phosphorsäure, Schwefelsäure, Bromwasserstoffsäure, Jodwasserstoffsäure, salpetrige Säure oder phosphorige Säure, oder organische Säuren, wie beispielsweise aliphatische Mono- oder Dicarbonsäuren, phenylsubstituierte Alkancarbonsäure, Hydroxyalkancarbonsäuren oder Alkandicarbonsäuren, aromatische Säuren oder aliphatische oder aromatische Sulfonsäuren. Physiologisch unbedenkliche Salze dieser Säuren sind daher z.B. das Sulfat, Pyrosulfat, Bisulfat, Sulfit, Bisulfit, Nitrat, Phosphat, Monohydrogenphosphat, Dihydrogenphosphat, Metaphosphat, Pyrophosphat, Chlorid, Bromid, Jodid, Fluorid, Acetat, Propionat, Decanoat, Caprylat, Acrylat, Formiat, Isobutyrat, Caproat, Heptanoat, Propiolat, Malonat, Succinat, Suberat, Sebacat, Fumarat, Maleat, Mandelat, Butin-1.4-dioat, Hexin-1.6-dioat, Benzoat, Chlorbenzoat, Methylbenzoat, Dinitrobenzoat, Hydroxybenzoat, Methoxybenzoat, Phthalat, Terephthalat, Benzolsulfonat, Toluolsulfonat, Chlorbenzolsulfonat, Xylolsulfonat, Phenylacetat, Phenylpropionat, Phenylbutyrat, Citrat, Lactat, β-Hydroxybutyrat, Glycollat, Malat, Tartrat, Methansulfonat, Propansulfonat, Naphthalin-1-sulfonat oder Naphthalin-2-sulfonat.

Die Alkylreste mit bis zu 4 C-Atomen sind solche, die sich von den aliphatischen Kohlenwasserstoffen ableiten, wie z.B. Methyl, Ethyl, n-Propyl, Isopropyl, n-Butyl, Isobutyl und tert.-Butyl.

Bildet der Substituent R gemeinsam mit dem Aminostickstoff einen Ring, so hat dieser 2-8 Kohlenstoffatome und stellt z.B. ein Aziridin-, Pyrrolidin- oder Piperidinringsystem dar.

Unter Acyl sind Saurereste wie Alkanoyl mit bis zu5 C-Atomen, Aroyl und Aralkanoyl mit 7-10 C-Atomen zu verstehen.

Die Alkanoylreste mit 1 bis 5 C-Atomen leiten sich ab von aliphatischen Carbonsäuren, wie z.B. Acetyl, Propanoyl, n-Butanoyl und i-Butanoyl.

Die Aroylreste und Aralkanoylreste mit 7 bis bis 10 C-Atomen sind beispielsweise Benzoyl-, p-Methylbenzoyl, 3,5-Dimethylbenzoyl, Phenylacetyl, Phenylpropanoyl und p-Tolylacetyl.

Als $C_{1-4}$-Alkyl- und $C_{6-8}$-Arylsulfonylreste seien beispielsweise genannt der Methansulfonyl- und der p-Toluolsulfonylrest.

Halogen im Sinne dieser Anmeldung bedeutet vorzugsweise Chlor und Brom.

Ergoline mit Substituenten im aromatischen Teil des Moleküls, d.h. in der Position 12, 13 oder 14, sind

z.B. aus DE OS 2 601 473, DE OS 3 205 169, EP-A-48695 und GB-A-2093452 bekannt. In 12-, 13- und 14-Stellung unsubstituierte Ergolinylharnstoffderivate werden beispielsweise in EP-A-74921 und im Merck-Index, 10th ed., 1983, S. 791 beschrieben.

Die erfindungsgemäßen 9.10-Didehydro-ergolinylharnstoff-Derivate zeigen im Vergleich zum 3-(9.10-Didehydro-6-methyl-8α-ergolinyl)-1.1-diethylharnstoff eine schwächer ausgeprägte oder fehlende zentral dopaminerge Wirkung (Parameter: Stereotypien), stärker ausgeprägte adrenerge Stimulationszeichen (Parameter: Piloerektion) und damit verbunden schwächere bzw. fehlende adrenolytische Wirkungen (Parameter: Hautrötung).

Diese Verbindungen wären daher beispielsweise als Geriatrika, Antidepressiva und eventuell auch als Antihypertensiva therapeutisch anwendbar.

Die erfindungsgemäßen Ergolinylharnstoff-Derivate zeigen im Vergleich zum 3-(6-Methyl-8α-ergolinyl)-1.1-diethylharnstoff eine schwächer ausgeprägte adrenolytische Wirkung (Parameter: Hautrötung), eine stärker ausgeprägte kataleptogene Wirkung und einen stärker ausgeprägten hypothermen Effekt.

Die Verbindungen wären daher beispielsweise als Neuroleptika mit weniger ausgeprägten extrapyramidalen und neuroendokrinen Nebenwirkungen und zur Behandlung extrapyramidalmotorischer Bewegungsanomalien (Dystonien, tardive Dyskinesien, Morbus Huntington) geeignet.

Die pharmakologische Wirksamkeit der erfindungsgemäßen Ergolinylharnstoff- bzw. 9.10-Didehydroergolinylharnstoff-Derivate wurden in ausgewählten Parametern des Maus-Screen (mod. n. Irwin, S., Psychopharmacologia 13:227-257, 1968) getestet. Es wurde die niedrigste voll wirksame Dosis (Wirkung bei mindestens 2 von 3 Tieren) nach einmaliger i.p. Verabreichung in einem der erwähnten Parameter ermittelt.

Zur Verwendung der erfindungsgemäßen Verbindungen als Arzneimittel werden diese in die Form eines pharmazeutischen Präparats gebracht, das neben dem Wirkstoff für die enterale oder parenterale Applikation geeignete pharmazeutische, organische oder anorganische inerte Trägermaterialien, wie z.B. Wasser, Gelatine, Gummi arabicum, Milchzucker, Stärke, Magnesiumstearat, Talk, pflanzlische Öle, Polyalkylenglykole usw. enthält. Die pharmazeutischen Präparate können in fester Form, z.B. als Tabletten, Dragées, Suppositorien, Kapseln oder in flüssiger Form, z.B. als Lösungen, Suspensionen oder Emulsionen vorliegen. Gegebenenfalls enthalten sie darüber hinaus Hilfsstoffe wie Konservierungs-, Stabilisierungs-, Netzmettel oder Emulgatoren, Salze zur Veränderung des osmotischen Druckes oder Puffer.

Die Dosierung der erfindungsgemäßen Verbindungen liegt beim Menschen im Bereich von 5-100 mg/Tag und eine Dosierungsform enthält 1-20 mg Wirkstoff.

Die Herstellung der erfindungsgemäßen Verbindungen erfolgt nach an sich bekannten Methoden.

Die Erfindung betrifft das Verfahren zur Herstellung von substituierten Ergolinen der allgemeinen Formel I dadurch gekennzeichnet, daß man ein im aromatischen Ring des Ergolinmoleküls unsubstituiertes Ergolin der allgemeinen Formel II

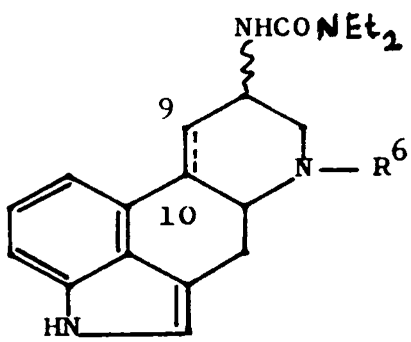

(II),

worin die 8-ständige Harnstoffseitenkette α- oder β-ständig sein kann,

$C_9$---$C_{10}$ eine CC-Einfach- oder C=C-Doppelbindung bedeuten und $R^6$ die oben angegebene Bedeutung haben,

mit Natriumborhydrid in Trifluoressigsäure zur 2.3-Dihydroverbindung reduziert, gegebenenfalls in 1-Stellung acyliert, mit Salpetersäure nitriert, gegebenenfalls die erhaltene Nitroverbindung in 1-Stellung verseift oder

alkyliert und anschließend die Nitrogruppe zur Aminogruppe reduziert

oder gegebenenfalls die erhaltene Nitroverbindung in 1-Stellung verseift und in 2.3-Stellung dehydriert und die so erhaltene 2.3-dehydrierte Nitroverbindung am 1-ständigen Stickstoff gegebenenfalls acyliert, sulfonyliert oder alkyliert und anschließend die Nitrogruppe zur Aminogruppe reduziert oder die oben erhaltene 2.3-dehydrierte Nitroverbindung in 2-Stellung halogeniert und anschließend die Nitrogruppe zur Aminogruppe reduziert und gegebenenfalls eine so erhaltene Aminoverbindung alkyliert oder acyliert und gegebenenfalls die so erhaltene Acylverbindung zur Mono-Alkylverbindung reduziert,
und gegebenenfalls anschließend in ein Säureadditionssalz überführt.

Das verwendete Ausgangsmaterial der Formel II ist bekannt. Es sind Ergolin-diethylharnstoff-Derivate, bei denen die Ureido-Seitenkette entweder $\alpha$- oder $\beta$-ständig ist, die in der 9.10-Stellung gesättigt oder ungesättigt und am Stickstoffatom in 6-Stellung mit $C_{1-4}$-Alkyl substituiert sind (z.B. DE OS 1.695.771, DE PS 2.238.540, DE OS 2.924.102).

Das Indol-System dieser Harnstoff-Derivate der Formel II wird mit Natriumborhydrid in Trifluoressigsäure in 2.3-Stellung selektiv zum Indolin-System reduziert (DE OS 2.810.774) und gewünschtenfalls in 1-Stellung mit einem reaktiven Säurederivat wie Essigsäureanhydrid oder Acetylchlorid in Pyridin acyliert.

Die Nitrierung erfolgt mit Salpetersäure in Gegenwart von Schwefelsäure oder Essigsäure, wobei die Reaktion bei Verwendung überschüssiger Salpetersäure neben der gewünschten Nitrierung in 12-, 13- und 14-Stellung, ggf. geringe Mengen an 1-Nitro- und/oder 9-Nitroverbindungen liefert. Die Mono-Nitrierung erfolgt im Wesentlichen nach den Regeln der elektrophilen Substitution von Anilin, wobei die 2.3-Dihydroergoline in 13-Stellung, die acylierten 2.3-Dihydro-ergoline und die 9.10-Didehydro-2.3-dihydro-ergoline in 12- und 14-Stellung nebeneinander hauptsächlich nitriert werden. Darüberhinaus können kleine Mengen der übrigen Stellungsisomeren und doppelt nitrierte Produkte entstehen. Die Trennung erfolgt durch Chromatographie oder Kristallisation.

Enthält die so erhaltene Nitroverbindung in 1-Stellung eine Acylgruppe, so wird diese durch verdünnte Mineralsäure, wie In Salzsäure oder mit Hydrazin verseift.

Die in 1-Stellung unsubstituierte Nitroverbindung wird anschließend wieder oxidativ in das Ergolin- bzw. Indolsystem überführt. Hierzu wird diese Verbindung in einem inerten Lösungsmittel, wie chlorierte Kohlenwasserstoffe, mit einem Oxydationsmittel wie Braunstein, Nickelperoxid, Derivate der Chromsäure, Phenylselenigsäureanhydrid` Palladiumsalze, Sauerstoff und Katalysatoren oder mit Dimethylsulfid, tert.-Butylhypochlorit und Base, umgesetzt.

Gewünschtenfalls kann die zuvor genannte Verbindung in 1-Stellung am Indolstickstoff wieder acyliert oder alkyliert werden. Die Alkylierung erfolgt nach Y. Kikugawa et al., Synthesis 1981, 461, die Acylierung und Sulfonylierung nach V.O. Illi, Synthesis 1975 387.

Anschließend kann die Nitrogruppe zur Aminogruppe selektiv, d.h. ohne Reduktion einer gegebenenfalls vorhandenen 9.10-Doppelbindung mit Natriumborhydrid in Gegenwart von Metallsalzen wie Nickel-(II)-Salzen oder Zinn-(II)-Salzen reduziert werden (A. Nose et al., Chem.Pharm.Bull. 29, 1155 (1981) und T. Satoh et al., Chem.Pharm.Bull. 29, 1443 (1981)).

Des weiteren kann aber auch die nitrierte Ergolinylharnstoffverbindung in 2-Stellung halogeniert werden. Hierzu wird das Ausgangsmaterial in einem inerten Lösungsmittel gelöst und mit einem Halogenierungsmittel umgesetzt.

Als Chlorierungsmittel eignen sich N.2.6-Trichlor-4-nitroacetanilid, N'-Chlor-succinimid, N-Chlorsaccharin, t-Butylhypochlorit, N-Chloracetanilid, N-Chlorphthalimid, N-Chlortetrachlorphthalimid, 1-Chlorbenzotriazol, N-Chlor-2.4.6-trichloracetanilid, Thionylchlorid, Sulfurylchlorid, Sulfurylchlorfluorid, Cyanurtrichlorid, Kupfer-II-chlorid, Hexachloraceton, Tetraalkylammoniumperchlorid wie Tetramethylammoniumperchlorid und Natriumhypochlorit.

Zur Bromeinführung eignen sich N-Bromsuccinimid sowie Brom, N-Brom-acetamid, N-Bromphthalimid, N.N-Dibromhydantoin, N-Brom-p-toluolsulfamid, N-Brom-di-p-toluol-sulfimid, Pyrrolidon-(2)-hydrotribromid, N-Bromcaprolactam, Dioxandibromid, Pyridiniumbromid, Pyridiniumperbromid, Phenyltrimethylammoniumbromid, Phenyltrimethylammoniumperbromid, 3-Brom-6-chlor-2-methyl-imidazo[1.2-b]-pyridazin als Bromkomplex, Kupfer-II-bromid, Natriumhypobromid, 5.5-Dibrom-2.2-dimethyl-4.6-dioxo-1.3-dioxan, 2.4.4.6-Tetrabrom-cyclohexa-2.5-dienon, 2-Carboxyethyltriphenylphosphonium-perbromid, Tetraalkylammoniumperbromid, wie Tetramethylamminium-perbromid und 1.3-Dibrom-5.5-dimethylhydantoin.

Die Halogenierungsmittel können in verschiedenen Lösungsmitteln eingesetzt werden. Geeignete Lösungsmittel sind immer solche, die gegenüber den Reaktanten inert sind. Genannt seien beispielsweise aliphatische und cyclische Ether wie Diethylether, Methylethylether, Tetrahydrofuran und Dioxan, halogenierte Kohlenwasserstoffe wie Methylenchlorid, Chloroform und Tetrachlorkohlenstoff, polare aprotische Lösungsmitel wie Hexamethylphosphortriamid, Acetonitril, Dimethylformamid, N-Methylpyrrolidon, Dimethylacetamid und Tetramethylharnstoff, gesättigte und ungesättigte Carbocyclen wie Hexan, Benzol und Toluol sowie Ketone wie Aceton, Cyclohexanon, Methylethylketon und Methylisobutylketon.

Die Losungsmittel können einzeln oder im Gemisch untereinander angewendet werden.

Gewünschtenfalls kann die zur Aminoverbindung reduzierte Nitroverbindung durch Alkylierung und

Acylierung zu den entsprechenden Alkyl- bzw. Acylverbindungen umgesetzt werden. Eine so erhaltene Acylaminoverbindung kann gewünschtenfalls durch Reduktion mit Di-isobutylaluminiumhydrid, mit Lithiumaluminiumhydrid oder mit Borandimethylsulfid in die entsprechenden Monoalkylaminoverbindungen umgesetzt werden.

Das erfindungsgemäße Verfahren verläuft nach folgendem Formelschema:

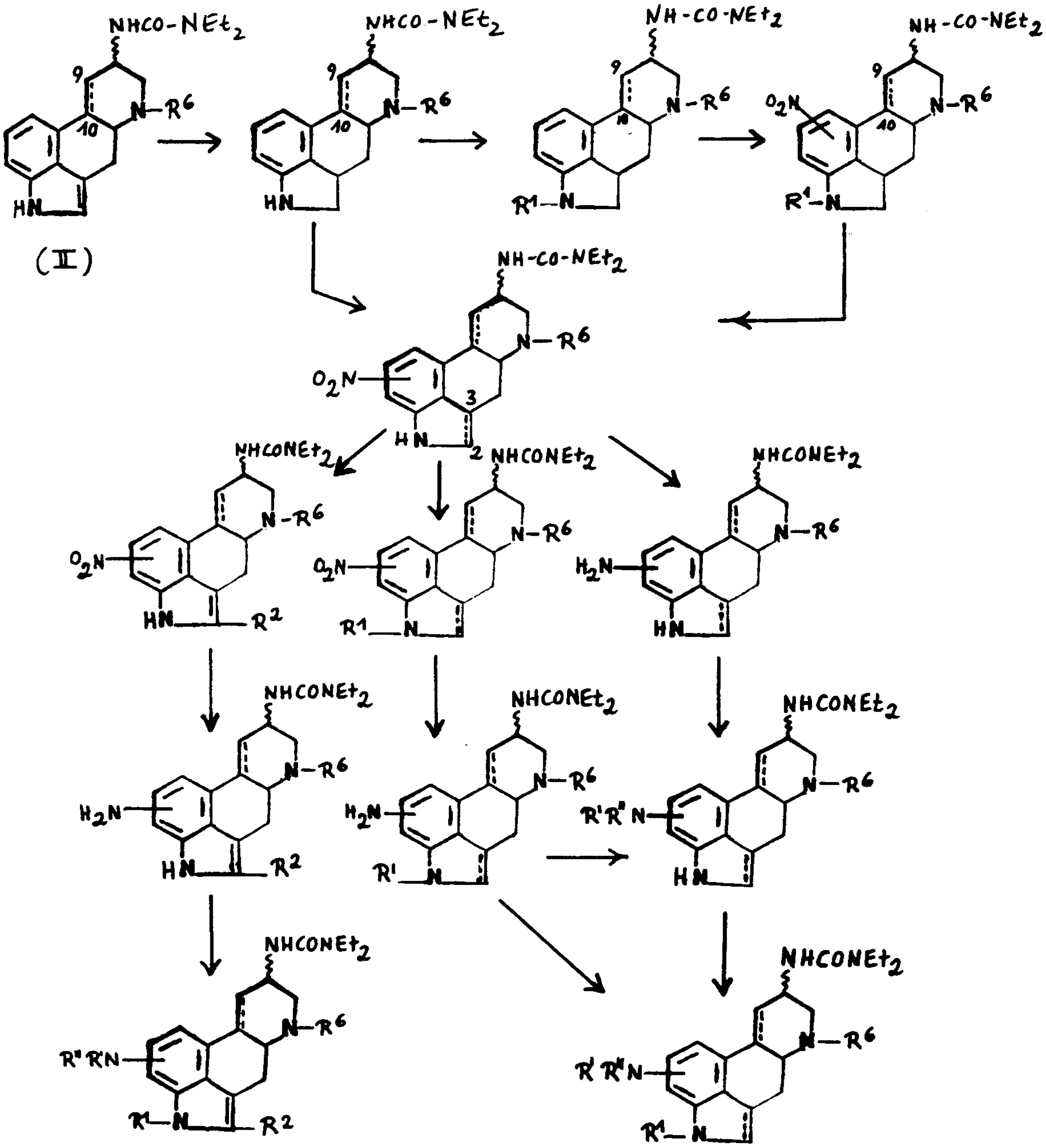

Die so erhaltenen Verbindungen werden entweder als freie Basen oder in Form ihrer Säureadditionssalze, die gewünschtenfalls durch Umsetzung mit einer physiologisch verträglichen Säure, wie z.B. Weinsäure, Maleinsäure oder Benzoesäure, erhalten werden, durch Umkristallisation und/oder Chromatographie gereinigt.

Zur Bildung von Salzen wird die erhaltene Verbindung in wenig Methanol gelöst und mit einer konzentrierten Lösung der gewünschten organischen Säure in Methanol bei Raumtemperatur versetzt.

Beispiel 1

Man löst 19,6 g 3-(9.10-Didehydro-6-methyl-8α-ergolinyl)-1.1-diethylharnstoff bei -20 °C in 80 ml

Trifluoressigsäure und gibt in Abständen von 3-4 Minuten 8 Portionen von jeweils 500 mg Natriumborhydrid zu. Anschließend schüttet man die Mischung auf Eis, macht unter Eiskühlung mit 90 ml 26%iger Ammoniaklösung alkalisch und extrahiert mit Methylenchlorid. Die organische Phase wird getrocknet, eingeengt und an Kieselgel mit Methylenchlorid/Methanol chromatographiert. Man isoliert 10,6 g 3-(9.10-Didehydro-2.3-dihydro-6-methyl-8α-ergolinyl)-1.1-diethylharnstoff als Isomerengemisch der 3α- und 3β-H-Verbindungen. Durch Kristallisation aus Essigester werden 6,5 g der 3β-H-Verbindung erhalten.
$[\alpha]_D$ = +207 ° (0,5 % in Chloroform).

Die Mutterlauge ist im wesentlichen eine Mischung der 3α- und 3β-H isomeren Verbindungen und meist als Gemisch für weitere Reaktionsstufen einsetzbar.

In gleicher Weise werden aus den entsprechenden 2.3-ungesättigten Ergolinen die folgenden hydrierten 2.3-Dihydro-Verbindungen dargestellt:
3-(2.3-Dihydro-6-methyl-8α-ergolinyl)-1.1-diethylharnstoff
$[\alpha]_D$ = +44 ° (0,5 % in Chloroform), Ausbeute 69 %, davon 30 % als kristalline 3β-H-Verbindung:
3-(2.3-Dihydro-6-methyl-8α-ergolinyl)-1.1-diethylharnstoff-weinsaures Salz (1:1)
$[\alpha]_D$ = +31 ° (0,5 % in Pyridin);
3-(9.10-Didehydro-2` 3β-dihydro-6-methyl-8β-ergolinyl)-1.1-diethylharnstoff;
$[\alpha]_D$ = +10 ° (0,5 % in $CHCl_3$)
3-(2.3-Dihydro-6-methyl-8β-ergolinyl)-1.1-diethylharnstoff, isoliert als Isomerengemisch (3α und 3βH);
3-(2.3-Dihydro-6-n-propyl-8α-ergolinyl)-1.1-diethylharnstoff; $[\alpha]_D$ = +14 ° (0,5 % in $CHCl_3$);

## Beispiel 2

Man löst 1 mMol 3-(9.10-Didehydro-2.3β-dihydro-6-methyl-8α-ergolinyl)-1.1-diethylharnstoff in 2 ml Pyridin und 2 ml Acetanhydrid, läßt bei Raumtemperatur 2 Stunden stehen und gibt die Mischung auf Eis. Nach 15 Minuten Rühren macht man mit verdünnter Ammoniaklösung alkalisch und extrahiert mit Methylenchlorid, trocknet und dampft ein.

Man erhält in einer Ausbeute von 96 % 3-(1-Acetyl-9.10-didehydro-2.3-dihydro-6-methyl-8α-ergolinyl)-1.1-diethylharnstoff als kristalline 3β-H-Verbindung.
$[\alpha]_D$ = +190 ° (0,5 % in Chloroform).

In gleicher Weise werden aus den 2.3-Dihydroergolin-Derivaten die folgenden 1-Acetyl-2.3-dihydroergolin-Derivate dargestellt:
3-(1-Acetyl-2.3α-dihydro-6-methyl-8α-ergolinyl)-1.1-diethylharnstoff
Ausbeute 85 %.
$[\alpha]_D$ = +46 ° (0,5 % in Chloroform);
3-(1-Acetyl-2.3β-dihydro-6-methyl-8α-ergolinyl)-1.1-diethylharnstoff
$[\alpha]_D$ = -61 ° (0,5 % in Chloroform);

## Beispiel 3

Man löst 10 mMol 3-(2.3-Dihydro-6-methyl-8α-ergolinyl)-1.1-diethylharnstoff in 100 ml konzentrierter Schwefelsäure unter Eiskühlung, gibt dazu 7 ml einer Lösung von 10 ml 65 %iger Salpetersäure in 90 ml konzentrierter Schwefelsäure und rührt 15 Minuten im Eisbad weiter. Dann tropft man die Reaktionslösung langsam in Eis ein, macht unter Eiskühlung mit 32 %iger Ammoniaklösung alkalisch und schüttelt mit Methylenchlorid aus. Nach Waschen der organischen Phase mit Wasser und Trocknen mit Magnesiumsulfat erhält man die nitrierte Verbindung in 90 %iger Rohausbeute. Nach chromatographischer Auftrennung bzw. Reinigung erhält man 3-(2.3-Dihydro-6-methyl-13-nitro-8α-ergolinyl)-1.1-diethylharnstoff in einer Ausbeute von 71 %.
$[\alpha]_D$ = +52 ° (0,5 % in Chloroform).

## Beispiel 4

In analoger Weise werden durch Nitrierung der entsprechenden in 12-, 13- und 14-Stellung unsubstituierten Ergolinderivate die folgenden Verbindungen erhalten:

I. 3-(1-Acetyl-2.3-dihydro-6-methyl-12-nitro-8α-ergolinyl)-1.1-diethylharnstoff als Isomerengemisch der 3α- und 3β-H-Verbindungen.

II. 3-(9.10-Didehydro-2.3β-dihydro-6-methyl-12-nitro-8α-ergolinyl)-1.1-diethylharnstoff;
$[\alpha]_D$ = -674 ° (0,5 % in $CHCl_3$)
Ausbeute: 50 %.

III. 3-(9.10-Didehydro-2.3-dihydro-6-methyl-14-nitro-8α-ergolinyl)-1.1-diethylharnstoff,
Ausbeute: 17 %;
IV. 3-(2.3β-Dihydro-6-methyl-13-nitro-8β-ergolinyl)-1.1-diethylharnstoff; Ausbeute 53 %
$[\alpha]_D$ = +70 ° (0,5 % in ln HCl)
3-(2.3α-Dihydro-6-methyl-13-nitro-8β-ergolinyl)-1.1-diethylharnstoff; Ausbeute 15 %, $[\alpha]_D$ = - 150 ° (0,5 % in $CHCl_3$);
V. 3-(9.10-Didehydro-2.3β-dihydro-6-methyl-12-nitro-8β-ergolinyl)-1.1-diethylharnstoff;
Ausbeute: 30 %, $[\alpha]_D$ = -593 ° (0,5 % in MeOH) als weinsaures Salz
VI. 3-(2.3α-Dihydro-13-nitro-6-n-propyl-8α-ergolinyl)-1.1-diethylharnstoff, Ausbeute: 50 %
$[\alpha]_D$ = -156 ° (0,5 % in $CHCl_3$)
3-(2.3β-Dihydro-13-nitro-6-n-propyl-8α-ergolinyl)-1.1-diethylharnstoff, Ausbeute: 38 %
$[\alpha]_D$ = +86 ° (0,5 % in $CHCl_3$).

Beispiel 5

Man erwärmt 1 mMol 3-(1-Acetyl-2.3β-dihydro-6-methyl-12-nitro-8α-ergolinyl)-1.1-diethylharnstoff in einer Mischung von 2 ml wasserfreiem Hydrazin und 2 ml Chloroform 3 Stunden auf 50 °C, gießt in Wasser ein, extrahiert mit Methylenchlorid, trocknet die organische Phase und dampft im Vakuum ein. Man erhält 230 mg 3-(2.3-Dihydro-6-methyl-12-nitro-8α-ergolinyl)-1.1-diethylharnstoff.
Ausbeute: 25 %
$[\alpha]_D$ = +538 ° (0,5 % in Methanol) als weinsaures Salz.

Beispiel 6

1 mMol 3-(1-Acetyl-2.3-dihydro-6-methyl-12-nitro-8α-ergolinyl)-1.1-diethylharnstoff gelöst in 5 ml ln Salzsäure wird 1 Stunde auf 95 °C erwärmt. Nach Abkühlung wird mit verdünnter Ammoniaklösung alkalisch gemacht, mit Methylenchlorid versetzt und extrahiert. Nach Trocknen der organischen Phase wird eingedampft. Man erhält 220 mg 3-(2.3-Dihydro-6-methyl-12-nitro-8α-ergolinyl)-1.1-diethylharnstoff.

Beispiel 7

Man löst l mMol 3-(2.3-Dihydro-6-methyl-13-nitro-8α-ergolinyl)-1.1-diethylharnstoff in 10 ml Methylenchlorid und gibt 2 g Braunstein in fester Form zu. Dann rührt man 2 Stunden bei Raumtemperatur. Nach Abfiltrieren des überschüssigen Braunsteins wird das Filtrat eingedampft. Der Rückstand wird an Kieselgel mit Methylenchlorid/Methanol (5 %) chromatographiert. Man erhält 210 mg 3-(6-Methyl-13-nitro-8α-ergolinyl)-1.1-diethylharnstoff.
$[\alpha]_D$ = -2 ° (0,5 % in Chloroform).

In analoger Weise werden aus den entsprechenden 2.3-Dihydro-ergolinen die folgenden Verbindungen hergestellt:
1.1-Diethyl-3-(6-methyl-12-nitro-8α-ergolinyl)-harnstoff, Ausbeute: 50 %
3-(9.10-Didehydro-6-methyl-12-nitro-8α-ergolinyl)-1.1-diethylharnstoff, Ausbeute: 49 %, $[\alpha]_D$ = -344 ° (0,2 %
3-(9.10-Didehydro-6-methyl-14-nitro-8α-ergolinyl)-1.1-diethylharnstoff, Ausbeute: 30 %
$[\alpha]_D$ = +336 ° (0,5 % in Chloroform)
1.1-Diethyl-3-(6-methyl-13-nitro-8β-ergolinyl)-harnstoff, Ausbeute: 46 %, $[\alpha]_D$ = +4,5 ° (0,5% in $CHCl_3$);
3-(9.10-Didehydro-6-methyl-12-nitro-8β-ergolinyl)-1.1-diethylharnstoff, Ausbeute: 73 %,
$[\alpha]_D$ = -646 ° (0,5 % in $CHCl_3$);
1.1-Diethyl-3-(13-nitro-6-n-propyl-8α-ergolinyl)-harnstoff, Ausbeute: 70 %,
$[\alpha]_D$ = +2 ° (0,5 % in $CHCl_3$).

Beispiel 8

Man löst 150 mg 3-(9.10-Didehydro-2.3-dihydro-6-methyl-14-nitro-8α-ergolinyl)-1.1-diethylharnstoff in 5 ml Methylenchlorid, gibt 250 mg Pyridiniumdichromat gelöst in 2 ml Methylenchlorid zu und rührt 1 Stunde bei Raumtemperatur. Dann wird mit Wasser verdünnt, mit Methylenchlorid ausgeschüttelt, die organische Phase getrocknet und eingedampft.Der Rückstand wird an Kieselgel mit Methylenchlorid/Methanol (5%) chromatographiert. Man erhält 70 mg 3-(9.10-Didehydro-6-methyl-14-nitro-8α-ergolinyl)-1.1-diethylharnstoff.

Beispiel 9

Man gibt eine Lösung von 0,4 mMol tert.-Butylhypochlorit in 2 ml Methylenchlorid tropfenweise zu einer Lösung von 0,3 mMol 3-(2.3-Dihydro-6-methyl-13-nitro-8β-ergolinyl)-1.1-diethylharnstoff, 0,4 mMol Dimethylsulfid und 0,3 mMol Triethylamin in 5 ml Methylenchlorid bei -70 °C und rührt 2 Stunden. Dann gibt man die Lösung von 2 mMol Natriumethylat in 2 ml Ethanol innerhalb von 10 Minuten hinzu und läßt die Mischung innerhalb von 2 Stunden auf Raumtemperatur erwärmen. Dann versetzt man mit Wasser, extrahiert mit Methylenchlorid, trocknet die organische Phase und dampft ein. Nach Chromatographie an Kieselgel mit Methylenchlorid/Methanol (5%) erhält man 30 mg 3-(6-Methyl-13-nitro-8β-ergolinyl)-1.1-diethylharnstoff.

Beispiel 10

Man löst l mMol 3-(2.3β-Dihydro-6-methyl-12-nitro-8α-ergolinyl)-1.1-diethylharnstoff in 25 ml Methanol, kühlt im Eisbad und gibt 2 mMol Nickelchlorid (mit $6H_2O$) und dann 5 mMol Natriumborhydrid in 4-5 Portionen zu. Unter Eiskühlung säuert man mit 2n Salzsäure an, versetzt mit verdünnter Ammoniaklösung und schüttelt aus. Nach Trocknen und Eindampfen der organischen Phase erhält man 350 mg 3-(12-Amino-2.3-dihydro-6-methyl-8α-ergolinyl)-1.1-diethylharnstoff.
$[\alpha]_D$ = +31 ° (0,5 % Methanol) als weinsaures Salz (1:1).

In analoger Weise werden aus den entsprechenden Nitroverbindungen die folgenden Amino-ergoline erhalten:
3-(13-Amino-2.3-dihydro-6-methyl-8α-ergolinyl)-1.1-diethylharnstoff, Ausbeute: 64 %
3-(12-Amino-9.10-didehydro-2.3-dihyro-6-methyl-8α-ergolinyl)-1.1-diethylharnstoff, Ausbeute: 48 %
3-(1-Acetyl-13-amino-2.3-dihydro-6-methyl-8α-ergolinyl)-1.1-diethylharnstoff, Ausbeute: 81 %
$[\alpha]_D$ = +14 ° (0,5 % in Chloroform)
3-(1-Acetyl-12-amino-9.10-didehydro-2.3-dihydro-6-methyl-8α-ergolinyl)-1.1-diethylharnstoff, Ausbeute: 65 %
$[\alpha]_D$ = +111 ° (0,5 % in Chloroform)
3-(12-Amino-6-methyl-8α-ergolinyl)-1.1-diethylharnstoff, Ausbeute: 83 %
3-(13-Amino-6-methyl-8α-ergolinyl)-1.1-diethylharnstoff, Ausbeute: 68 %
$[\alpha]_D$ = 0 ° (0,5 % in Chloroform)
3-(12-Amino-9.10-didehydro-6-methyl-8α-ergolinyl)-1.1-diethylharnstoff, Ausbeute: 53 %:
daraus das weinsaure Salz (1:1), Ausbeute: 83 % $[\alpha]_D$ = -320 ° (0,5 % in Pyridin);
3-(14-Amino-9.10-didehydro-6-methyl-8α-ergolinyl)-1.1-diethylharnstoff, Ausbeute: 65 %;
$[\alpha]_D$ = -305 ° (0,5 % in $CHCl_3$).
3-(13-Amino-6-methyl-8β-ergolinyl)-1.1-diethylharnstoff Ausbeute 82 %, $[\alpha]_D$ = 0 ° (0,5 % in Pyridin) als weinsaures Salz
3-(13-Amino-1.6-dimethyl-8α-ergolinyl)-1.1-diethylharnstoff, Ausbeute 64 %, $[\alpha]_D$ = - 8° (0,5 % in $CHCl_3$);
3-(12-Amino-9.10-didehydro-6-methyl-8β-ergolinyl)-1.1-diethylharnstoff, Ausbeute 36 %,
$[\alpha]_D$ = -605 ° (0,5 % in MeOH);
3-(1-Acetyl-13-amino-6-methyl-8α-ergolinyl)-1.1-diethylharnstoff, Ausbeute 48 %,
$[\alpha]_D$ = -28 ° (0,5 % in $CHCl_3$);
3-(13-Amino-6-n-propyl-8α-ergolinyl)-1.1diethylharnstoff Ausbeute 80 %, $[\alpha]_D$ = -2 ° (0,5 % in $CHCl_3$);
3-(12-Amino-2-brom-9.10-didehydro-6-methyl-8α-ergolinyl1.1-diethylharnstoff, Ausbeute 63 %,
$[\alpha]_D$ = -305 ° (0,5 % in $CHCl_3$).

Beispiel 11

Man löst 355 mg 3-(13-Amino-6-methyl-8α-ergolinyl)-1.1-diethylharnstoff in 10 ml Tetrahydrofuran, gibt 0,5 ml Ethyl-diisopropyl-amin (Hünig-Base) und 1 ml Methyljodid zu und erhitzt 3 Stunden zum Sieden. Man dampft die Hauptmenge des Lösungsmittels ab, verteilt zwischen Methylenchlorid und verdünnter Ammoniaklösung, trennt die organische Phase ab und trocknet sie. Nach dem Eindampfen wird an Kieselgel mit Methylenchlorid und Methanol (5 %) chromatographiert. Man erhält 310 mg 1.1-Diethyl-3-(13-dimethylamino-6-methyl-8α-ergolinyl)-harnstoff.
Ausbeute 73 %.

Beispiel 12

353 mg 3-(12-Amino-9.10-didehydro-6-methyl-8α-ergolinyl)-1.1-diethylharnstoff löst man in 5 ml wasserfreiem Pyridin, gibt 5 ml Essigsäureanhydrid zu und läßt 2 Stunden bei Raumtemperatur stehen. Dann rührt man die Mischung in Eis ein, läßt wieder 15 Minuten stehen und verteilt zwischen Methylenchlorid und Bicarbonatlösung. Die organische Phase wird getrocknet, eingedampft und wie in Beispiel 9 chromatographiert. Man erhält 250 mg 3-(12-Acetylamino-9.10-didehydro-6-methyl-8α-ergolinyl)-1.1-diethylharnstoff durch Umkristallisation aus Essigester/Diisopropylether.
$[\alpha]_D$ = +226 ° (0,5 % in Methanol).

Beispiel 13

200 mg 3-(12-Acetylamino-9.10-didehydro-6-methyl-8α-ergolinyl)-1.1-diethylharnstoff werden in 10 ml Dioxan gelöst, mit 2 ml 20% Diisobutyl-aluminiumhydrid-Lösung in Toluol versetzt und 7 Stunden auf 100 °C erhitzt. Man läßt abkühlen, gibt unter Kühlung erst Wasser, dann 2n Salzsäure zu und macht die Lösung mit verdünntem Ammoniak alkalisch, nachdem man etwa 1 g Weinsäure zugesetzt hat. Man schüttelt mit Methylenchlorid aus, trocknet die organische Phase und dampft ein. Der Rückstand wird wie in Beispiel 7 chromatographiert. Man erhält 135 mg 3-(9.10-Didehydro-12-ethylamino-6-methyl-8α-ergolinyl)-1.1-diethylharnstoff.
$[\alpha]_D$ = +135 ° (015 % in $CHCl_3$).

Beispiel 14

355 mg 3-(13-Amino-6-methyl-8α-ergolinyl)-1.1-diethylharnstoff werden bei -20 °C mit dem gemischten Anhydrid von Essigsäure und Ameisensäure zur N-Formylverbindung umgesetzt und diese mit Boran-Dimethylsulfid reduziert. (S. Krishnamurty, Tetrahedron-Letters 1982, 3315). Nach Chromatographie erhält man 260 mg 1.1-Diethyl-3-(6-methyl-13-methylamino-8α-ergolinyl)-harnstoff.
N-Formylverbindung: Ausbeute 66 %, $[\alpha]_D$ = -25 °
(0,5 % in Methanol)
N-Methylverbindung: Ausbeute 74 %, $[\alpha]_D$ = -15 °
(0,5 % in $CHCl_3$).

Beispiel 15

Man löst 410 mg 3-(13-Nitro-6-methyl-8α-ergolinyl)-1.1-diethylharnstoff in 10 ml Methylenchlorid, gibt 40 mg Tetrabutylamonniumhydrogensulfat und 0,8 ml Acetylchlorid zu und übergießt damit 1 g pulverisiertes Kaliumhydroxid. Nach 1,5 Stunden Rühren bei Raumtemperatur verdünnt man mit Natriumbicarbonatlösung, trennt die organische Phase ab und trocknet mit Natriumsulfat. Nach Eindampfen und Chromatographie wie in Beispiel 9 angegeben erhält man 375 mg 3-(1-Acetyl-6-methyl-13-nitro-8α-ergolinyl)-1.1-diethylharnstoff.
$[\alpha]_D$ = -34 ° (0,5 % in $CHCl_3$).

Beispiel 16

Analog Beispiel 15 wird die Alkylierung mit Methyljodid in Tetrahydrofuran durchgeführt. Man erhält 335 mg 1.1-Diethyl-3-(1.6-dimethyl-13-nitro-8α-ergolinyl)-harnstoff.
$[\alpha]_D$ = -15 ° (0,5 % in $CHCl_3$).

Beispiel 17

500 mg 3-(9.10-Didehydro-6-methyl-12-nitro-8α-ergolinyl)-1.1-diethylharnstoff löst man in 10 ml Dioxan und gibt bei Raumtemperatur 650 mg Pyrrolidon-hydroperbromid in fester Form zu. Man rührt 4 Stunden, gibt 1 ml Aceton zu und zieht die Hauptmenge des Lösungsmittels ab. Der Rückstand wird zwischen Methylenchlorid und Bicarbonatlösung verteilt, die organische Phase getrocknet und eingedampft. Der Rückstand wird an Kieselgel mit Methylenchlorid und Methanol chromatographiert. Man erhält 110 mg 3-(2-Brom-9.10-didehydro-6-methyl-12-nitro-8α-ergolinyl)-1.1diethylharnstoff.
$[\alpha]_D$ = -300 ° (0,2 % in Methanol).

**Patentansprüche**

**1.** Substituierte Ergoline der allgemeinen Formel I

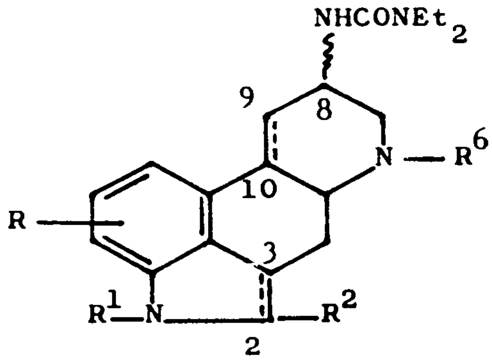

(I)

und deren Säureadditionssalze, worin die 8-ständige Harnstoff-Seitenkette $\alpha$-oder $\beta$-ständig sein kann, $C_2...C_3$ und $C_9...C_{10}$ eine CC-Einfach- oder C = C-Doppelbindung bedeuten und

- R Wasserstoff oder NR'R'', wobei R'R'' für $O_2$, $H_2$, $C_{1-4}$-Dialkyl steht oder gemeinsam mit dem Stickstoffatom einen 3- bis 9-gliedrigen Ring bildet oder R' und R'' jeweils allein für Wasserstoff, $C_{1-4}$-Alkyl oder $C_{1-10}$-Acyl stehen,
- $R^1$ für Wasserstoff, $C_{1-4}$-Alkyl, $C_{1-10}$-Acyl, $C_{6-8}$-Arylsulfonyl oder $C_{1-4}$-Alkylsulfonyl,
- $R^2$ für Wasserstoff oder Halogen, wenn $C_2...C_3$ eine C = C-Doppelbindung oder Wasserstoff, wenn $C_2...C_3$ eine CC-Einfachbindung darstellt und
- $R^6$ für $C_{1-4}$-Alkyl stehen und, wenn R ein Wasserstoffatom ist, $C_2...C_3$ eine CC-Einfachbindung darstellt.

**2.** 3-(9.10-Didehydro-6-methyl-12-nitro-8$\alpha$-ergolinyl)-1.1-diethylharnstoff

**3.** 3-(12-Amino-9.10-didehydro-6-methyl-8$\alpha$-ergolinyl)-1.1-diethylharnstofftartrat

**4.** 3-(6-methyl-13-nitro-8$\alpha$-ergolinyl)-1.1-diethylharnstoff

**5.** 3-(13-Amino-6-methyl-8$\alpha$-ergolinyl)-1.1-diethylharnstoff

**6.** Arzneimittel auf Basis der Verbindungen gemäß Anspruch 1-5.

**7.** Verfahren zur Herstellung von substituierten Ergolinen der allgemeinen Formel I

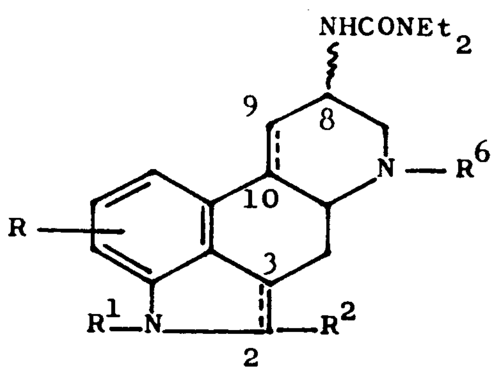

(I)

und deren Säureadditionssalze, worin die 8-ständige Harnstoff-Seitenkette $\alpha$-oder $\beta$-ständig sein kann, $C_2...C_3$ und $C_9...C_{10}$ eine CC-Einfach- oder C = C-Doppelbindung bedeuten und

- R Wasserstoff oder NR'R'', wobei R'R'' für $O_2$, $H_2$, $C_{1-4}$-Dialkyl steht oder gemeinsam mit dem Stickstoffatom einen 3- bis 9-gliedrigen Ring bildet oder R' und R'' jeweils allein für Wasserstoff, $C_{1-4}$-Alkyl oder $C_{1-10}$-Acyl stehen,
- $R^1$ für Wasserstoff, $C_{1-4}$-Alkyl, $C_{1-10}$-Acyl, $C_{6-8}$-Arylsulfonyl oder $C_{1-4}$-Alkylsulfonyl,
- $R^2$ für Wasserstoff oder Halogen, wenn $C_2...C_3$ eine C = C-Doppelbindung oder Wasserstoff, wenn $C_2...C_3$ eine CC-Einfachbindung darstellt und

$R^6$ für $C_{1-4}$-Alkyl stehen und, wenn R ein Wasserstoffatom ist, $C_2...C_3$ eine CC-Einfachbindung darstellt,

dadurch gekennzeichnet, daß man ein im aromatischen Ring des Ergolinmoleküls unsubstituiertes Ergolin der allgemeinen Formel II

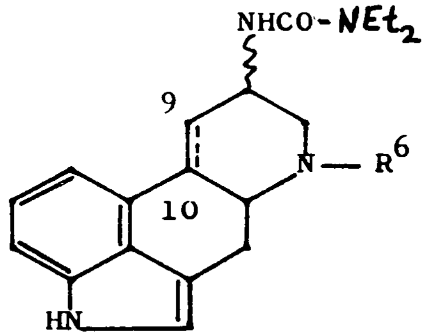

(II),

worin die 8-ständige Harnstoffseitenkette $\alpha$- oder $\beta$-ständig sein kann,

$C_9$---$C_{10}$ eine CC-Einfach- oder C=C-Doppelbindung bedeuten und $R^6$ die oben angegebene Bedeutung haben,

mit Natriumborhydrid in Trifluoressigsäure zur 2.3-Dihydroverbindung reduziert, gegebenenfalls in 1-Stellung acyliert, mit Salpetersäure nitriert, gegebenenfalls die erhaltene Nitroverbindung in 1-Stellung verseift oder

alkyliert und anschließend die Nitrogruppe zur Aminogruppe reduziert

oder gegebenenfalls die erhaltene Nitroverbindung in 1-Stellung verseift und in 2.3-Stellung dehydriert und die so erhaltene 2.3-dehydrierte Nitroverbindung am 1-ständigen Stickstoff gegebenenfalls acyliert, sulfonyliert oder alkyliert und anschließend die Nitrogruppe zur Aminogruppe reduziert oder die oben erhaltene 2.3-dehydrierte Nitroverbindung in 2-Stellung halogeniert und anschließend die Nitrogruppe zur Aminogruppe reduziert und gegebenenfalls eine so erhaltene Aminoverbindung alkyliert oder acyliert und gegebenenfalls die so erhaltene Acylverbindung zur Mono-Alkylverbindung reduziert,

und gegebenenfalls anschließend in ein Säureadditionssalz überführt.

## Claims

**1.** Substituted ergolines of the general formula I

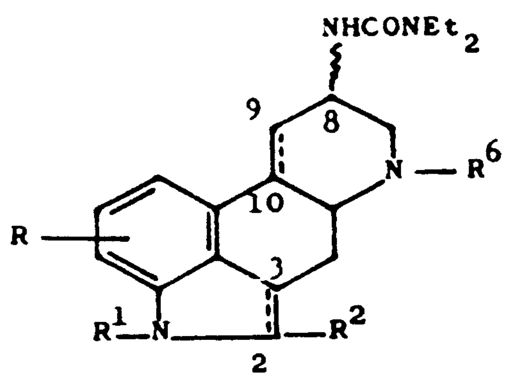

(I)

and the acid addition salts thereof, wherein the urea side chain in the 8-position may be in the $\alpha$- or $\beta$-configuration, $C_2$---$C_3$ and $C_9$---$C_{10}$ represent a CC single bond or C=C double bond and

R represents hydrogen or NR'R" in which R'R" represents $O_2$, $H_2$, $C_{1-4}$dialkyl or, together with the nitrogen atom, a 3- to 9-membered ring, or each of R' and R" represents hydrogen, $C_{1-4}$alkyl or $C_{1-10}$acyl,

$R^1$ represents hydrogen, $C_{1-4}$alkyl, $C_{1-10}$acyl, $C_{6-8}$arylsulphonyl or $C_{1-4}$alkylsulphonyl,

$R^2$ represents hydrogen or halogen when $C_2$---$C_3$ represents a C=C double bond, or hydrogen when $C_2$---$C_3$ represents a CC single bond, and

R⁶ represents $C_{1-4}$alkyl and, when R represents a hydrogen atom, $C_2$---$C_3$ represents a CC single bond.

2. 3-(9,10-Didehydro-6-methyl-12-nitro-8α-ergolinyl)-1,1-diethylurea.

3. 3-(12-Amino-9,10-didehydro-6-methyl-8α-ergolinyl)-1,1-diethylurea tartrate.

4. 3-(6-Methyl-13-nitro-8α-ergolinyl)-1,1-diethylurea.

5. 3-(13-Amino-6-methyl-8α-ergolinyl)-1,1-diethylurea.

6. Medicament based on the compounds according to claims 1 to 5.

7. Process for the manufacture of substituted ergolines of the general formula I

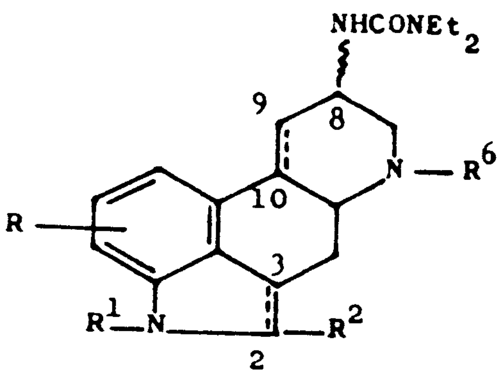

(I)

and the acid addition salts thereof, wherein the urea side chain in the 8-position may be in the α- or β-configuration, $C_2$---$C_3$ and $C_9$---$C_{10}$ represent a CC single bond or C=C double bond and represents hydrogen or NR'R" in which R'R" represents

R $O_2$, $H_2$, $C_{1-4}$dialkyl or, together with the nitrogen atom, a 3- to 9-membered ring, or each of R' and R" represents hydrogen, $C_{1-4}$alkyl or $C_{1-10}$acyl,

$R^1$ represents hydrogen, $C_{1-4}$alkyl, $C_{1-10}$acyl, $C_{6-8}$arylsulphonyl or $C_{1-4}$alkylsulphonyl,

$R^2$ represents hydrogen or halogen when $C_2$---$C_3$ represents a C=C double bond, or hydrogen when $C_2$---$C_3$ represents a CC single bond, and represents $C_{1-4}$alkyl and, when R represents a hydrogen

R⁶ atom, $C_2$---$C_3$ represents a CC single bond,

characterised in that an ergoline of the general formula II, unsubstituted in the aromatic ring of the ergoline molecule

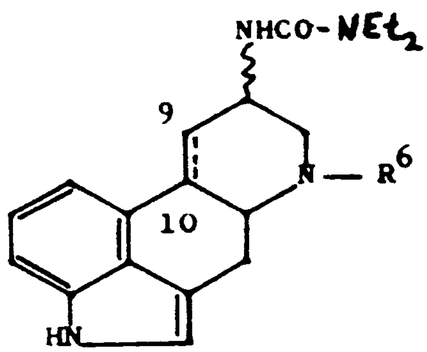

(II)

wherein the urea side chain in the 8-position may be in the α- or β-configuration,
$C_9$---$C_{10}$ represents a CC single bond or C=C double bond and $R^6$ is as defined above,
is reduced with sodium borohydride in trifluoroacetic acid to form the 2,3-dihydro compound, optionally

acylated in the 1-position, nitrated with nitric acid, the resulting nitro compound is optionally hydrolysed or alkylated in the 1-position and then the nitro group is reduced to the amino group, or optionally the resulting nitro compound is hydrolysed in the 1-position and dehydrogenated in the 2,3-position and the resulting 2,3-dehydrogenated nitro compound is optionally acylated, sulphonylated or alkylated at the nitrogen atom in the 1-position and then the nitro group is reduced to the amino group, or the above-obtained 2,3-dehydrogenated nitro compound is halogenated in the 2-position and then the nitro group is reduced to the amino group and optionally a resulting amino compound is alkylated or acylated and optionally the resulting acyl compound is reduced to the monoalkyl compound, and optionally an acid addition salt is formed.

## Revendications

**1.** Ergolines substituées de formule générale I et sels qu'elles forment par addition avec des acides :

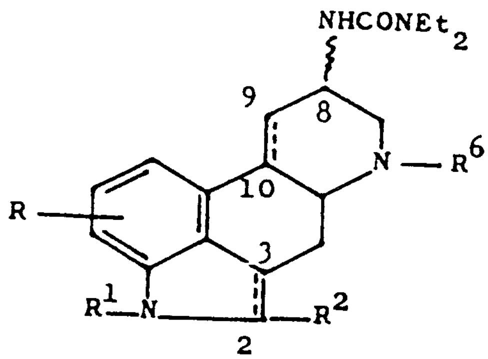

(I),

formule dans laquelle :
la chaîne latérale uréyle à la position 8 a la configuration $\alpha$ ou la configuration $\beta$,
$C_2...C_3$ et $C_9...C_{10}$ représentent chacun une liaison simple CC ou une double liaison C = C,

- R représente l'hydrogène ou un radical NR'R" dans lequel R'R" représente $O_2$, $H_2$ ou $C_{1-4}$-dialkyle ou R' et R" forment ensemble, et avec l'atome d'azote, un cycle comportant de 3 à 9 maillons, ou R' et R" représentent chacun isolément l'hydrogène, un alkyle en $C_1$-$C_4$ ou un acyle en $C_1$-$C_{10}$,
- $R^1$ représente l'hydrogène, un alkyle en $C_1$-$C_4$, un acyle en $C_1$-$C_{10}$, un arylsulfonyle en $C_6$-$C_8$ ou un alkylsulfonyle en $C_1$-$C_4$,
- $R^2$ représente l'hydrogène ou un halogène lorsque $C_2...C_3$ représente une double liaison C = C, ou l'hydrogène lorsque $C_2...C_3$ représente une liaison simple CC,
- $R^6$ représente un alkyle en C1-C4, et

lorsque R représente un atome d'hydrogène, $C_2...C_3$ représente une liaison simple CC.

**2.** (Didéhydro-9,10 méthyl-6 nitro-12 ergolinyl-8α)-3 diéthyl-1,1 urée.

**3.** Tartrate de l'(amino-12 didéhydro-9,10 méthyl-6 ergolinyl-8α)-3 diéthyl-1,1 urée.

**4.** (Méthyl-6 nitro-13 ergolinyl-8α)-3 diéthyl-1,1 urée.

**5.** (Amino-13 méthyl-6 ergolinyl-8α)-3 diéthyl-1,1 urée.

**6.** Médicament à base de composés selon l'une quelconque des revendications 1 à 5.

**7.** Procédé pour préparer des ergolines substituées de formule générale I et leurs sels d'addition d'acides :

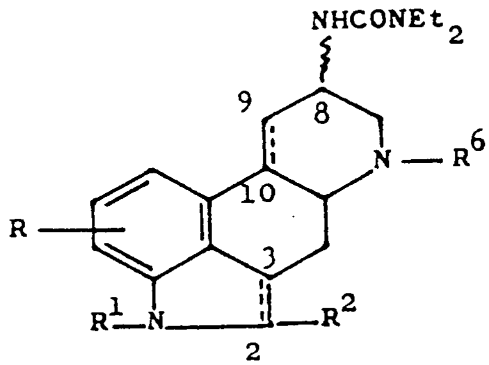

(I)

formule dans laquelle :
la chaîne latérale uréyle à la position 8 a la configuration α ou la configuration β,
$C_2...C_3$ et $C_9...C_{10}$ représentent chacun une liaison simple CC ou une double liaison C = C,

R représente l'hydrogène ou un radical NR'R" dans lequel R'R" représente $O_2$, $H_2$ ou $C_{1-4}$-dialkyle ou R' et R" forment ensemble, et avec l'atome d'azote, un cycle comportant de 3 à 9 maillons, ou R' et R" représentent chacun isolément l'hydrogène, un alkyle en $C_1$-$C_4$ ou un acyle en $C_1$-$C_{10}$,

$R^1$ représente l'hydrogène, un alkyle en $C_1$-$C_4$, un acyle en $C_1$-$C_{10}$, un arylsulfonyle en $C_6$-$C_8$ ou un alkylsulfonyle en $C_1$-$C_4$,

$R^2$ représente l'hydrogène ou un halogène lorsque $C_2...C_3$ représente une double liaison C = C, ou l'hydrogène lorsque $C_2...C_3$ représente une liaison simple CC,

$R^6$ représente un alkyle en C1-C4, et

lorsque R représente un atome d'hydrogène, $C_2...C_3$ représente une liaison simple CC,
procédé caractérisé en ce qu'on réduit une ergoline non substituée sur son cycle aromatique et répondant à la formule générale II :

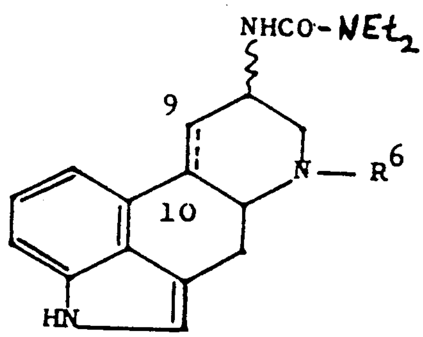

(II),

dans laquelle la chaîne uréyle latérale en 8 peut avoir la configuration α ou la configuration β, $C_9...C_{10}$ représente une liaison simple CC ou une liaison double C = C, et R6 a la signification qui lui a été donnée plus haut,
au moyen du tétrahydruroborate de sodium dans de l'acide trifluoracétique, pour obtenir le composé dihydro-2,3, on acyle éventuellement en position 1, on nitre par l'acide nitrique, éventuellement on saponifie ou alkyle en position 1 le composé nitré obtenu, puis on réduit le radical nitro en radical amino, ou éventuellement on saponifie en position 1 le composé nitré obtenu et on le déshydrogène en position 2,3, et éventuellement on acyle, sulfonyle ou alkyle à l'azote en 1 le composé nitré déshydrogéné en 2,3 ainsi obtenu, après quoi on réduit le radical nitro en radical amino, ou on halogène en position 2 le composé nitré déshydrogéné en 2,3 obtenu ci-dessus, puis on réduit le radical nitro en radical amino, et éventuellement on alkyle ou acyle un composé aminé ainsi obtenu, et éventuellement on réduit le composé acylique ainsi obtenu pour le convertir en éventuellement on transforme ensuite le composé obtenu en un sel d'addition d'acide.